# EUROPEAN PATENT APPLICATION

(11) **EP 3 254 708 A1**
(43) Date of publication of application: **13.12.2017**
(21) Application number: 16739820.5
(22) Date of filing: 13.01.2016
(51) Int. Cl.: A61L 9/12

(54) **AIR FRESHENER DEVICE FOR INTERIORS**

(30) Priority: 22.01.2015 ES 201530063 U
(71) Applicant: Garcia Carrasco, Manuel, 25600 Balaguer (Lérida) (ES); Garcia Oliva, Esther, 25600 Balaguer (Lérida) (ES)
(72) Inventor: GARCIA CARRASCO, Manuel, 25600 Balaguer (Lérida) (ES)
(74) Representative: Baños Treceño, Valentín
(86) International application number: PCT/ES2016/070009
(87) International publication number: WO 2016/116643

(57) **Abstract**

An air freshener device for interiors made up of a bottle closed by a lid, wherein the liquid or fragrance is inside the bottle, wherein the liquid can be refilled, the device being able to be placed on a piece of furniture or hung, and wherein it is particularly highlighted that the bottle is made of transparent blown glass and the lid, which is made of synthetic material, has a central and circular through bore or hole that is filled with an absorbent filter made of synthetic and ecological material that is soaked by a tilting action of the device, wherein the bottle has a secure closure of the assembly by means of threading.

## Description

### OBJECT OF THE INVENTION

The present specification defines an air freshener device for interiors made up of a transparent blown glass flask, inside of which a liquid fragrance or essence is housed, being covered by a lid made of wood or synthetic material that has a central through hole filled with an absorbent filter that enables the essence to filter through it and enables said essence to be released outside.

### BACKGROUND

The scope of the application of this invention is that of air freshener devices or systems or indoor air fresheners. In this regard, there are many different solutions in the market from the simple insertion of fragrance in an open container, to complex electric air fresheners, including air fresheners with the fragrance in sheets and with media such as, for example, those used in car air fresheners.

The present invention defines an air freshener for interiors, which is small in size, refillable and easy to use, which does not require media to be tilted or connected to a power source. It is true that there are devices that are somewhat related to that of the present invention. For example, there are air fresheners with lids that enable the evaporation of the fragrance through the lid, as in the registration ES1060347U owned by Pedro Font, which is made up of a completely porous lid, which means the fragrance evaporates quickly and, therefore, there is a high consumption of fragrance; the registration ES1060820U owned by Fernando Figueroa, with porous lids and elastic seals that enable a more controlled evaporation, although the lids require complex manufacturing for the purpose of safety; the registration ES1070150U owned by Victor Ribalda, which is similar to the previous one, but wherein the lid is a porous membrane rather than a solid material; ES1060008U owned by Antonio Alfonso Montiel, wherein a porous lid for air fresheners is also defined; or, for example, typical air fresheners with a lid with a through hole with a filtering element, as in that of the registration ES1070671 U owned by Victor Ribalda, which can be considered as the closest state of the art, which has the drawback that, due to the filtering element being in contact with the fragrance and having a large size and surface in contact with the outside, the consumption of the fragrance is very high with respect to the present invention.

The present invention resolves the problem of releasing fragrance to the outside in a controlled, effective and economical way, in a different and novel manner with respect to the current devices on the market, as well as enabling the emission to be controlled by means of a simple manual movement of the air freshener device, which is something that is not possible with current devices on the market. In addition, as shall be described below, due to the design thereof and the adjustment by threading, the prevention of possible spills can be guaranteed, as well as preventing the possibility of accessing the inside of the container containing the fragrance, thus avoiding possible accidents or poisonings due to incorrect handling.

### DESCRIPTION OF THE INVENTION

The present invention defines an air freshener device for interiors made up of a transparent bottle, preferably made of blown glass, although it may also be made of plastic material, which houses the fragrance, and said base is closed by threading of a lid made of wood or synthetic material of a plastic nature, with a central through hole filled with an absorbent filter. This air freshener is refillable, and in turn may have at the base thereof a disk support made of wood which is an accessory.

As stated previously, the lid is made of wood or synthetic material, which is an element that does not enable the fragrance to be released to the outside in a natural way, therefore and for said purpose, a through bore or hole is made, preferably circular in order to facilitate the production thereof, which is filled with an absorbent filter made up of synthetic and ecological material. This filter is not in direct contact with the liquid or fragrance, therefore its purpose is not to be constantly soaked with it and thus release it outside through capillarity, but rather its purpose is for the essence of the fragrance to be impregnated at a specific moment and released in a controlled, slow and homogeneous manner. In this way, fragrance saving is considerable without it affecting the final air freshener result in the outdoor space. Moreover, it must be noted that the central hole of the lid and specifically the diameter thereof may be variable. In turn, the lid may have an outer perimeter groove or slot that enables an improved handling of the lid.

This air freshener device, before being used for the first time, has the base thereof closed by a plastic plug that must be removed manually, and in order to do so, the lid made of wood or synthetic material must be unthreaded and said plug removed, and lastly, the bottle must be covered again with the lid. This implies that the closure between the lid and the bottle is by threading, and therefore, at the upper perimeter of the bottle and the inner face of the lid, there is an inner and outer thread that enables the advance, coupling and closure between both elements. This constitutes a configuration which guarantees that the liquid or fragrance is correctly contained inside the device and thus insures that there cannot be spills or undesired incidents due to incorrect handling of the air freshener, which is very important when children are involved.

Once it is to be used, it only needs to be manually tilted 180º in order for the filter of the lid to be impregnated with the essence. In this way, as previously stated, the absorbent filter releases the fragrance to the outside in a controlled, slow and homogeneous manner, giving the same air freshening results for a room, car or any indoor room, the consumption of the fragrance being much lower. If one wishes the essence in the environment to be more continuous and prolonged, the tilting function only needs to be repeated as many times as considered necessary, thereby increasing the intensity of the aroma and the duration thereof.

Lastly, it must be noted that the device is intended to be placed on a surface such as a table, piece of furniture or similar, but there is the possibility that the air freshener can be hung, for which a hooking mechanism to a rope, string or similar must be added, and care must be taken no to puncture the thickness of the lid, creating an uncontrolled fragrance opening or outlet.

To complete the present description, and for the purpose of aiding in a better understanding of the characteristics of the invention, a set of drawings is attached as an integral part thereof, wherein, by way illustration and not limitation, the following has been represented:
Fig. 1 is a perspective representation of the assembly of the air freshener device.
Fig. 2 is a perspective representation of the outer portion of the lid.
Fig. 3 is a perspective representation of the inner portion of the lid.
Fig. 4 is a perspective representation of the absorbent filter.
Fig. 5 is a cross section of the assembly of the air freshener device.

### DESCRIPTION OF THE DRAWINGS

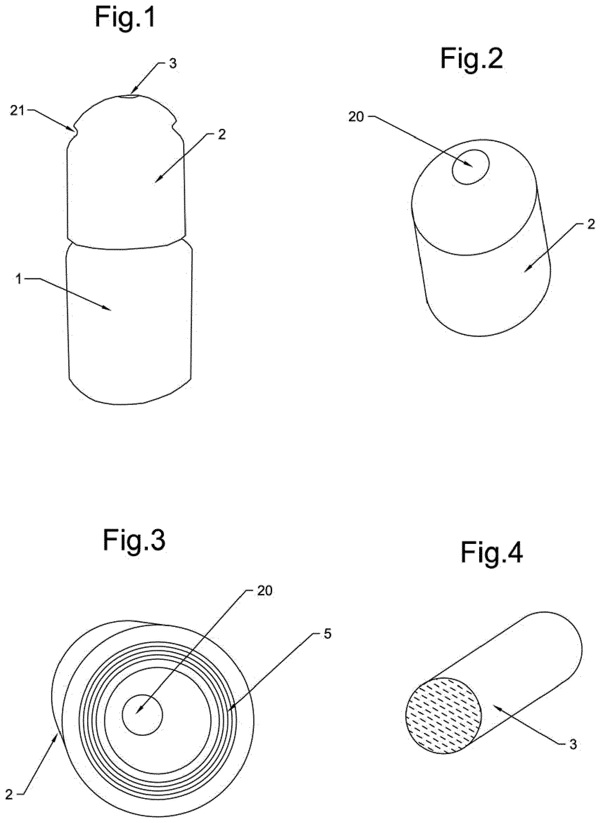

As can be seen in Figure 1, the air freshener device for interiors is made up of a bottle (1) closed by threading with a solid lid (2) made of wood or synthetic material with a central through hole (20) filled with an absorbent filter (3) such that said filter releases the fragrance in a controlled, slow and homogeneous manner, giving the same air freshening results for a room, car or any indoor room. The air freshener is refillable due to being able to be unthreaded. In this figure, it must be noted how the lid can have an outer perimeter groove or slot (21) that enables an improved manipulation of the lid.

Figures 2 and 3 show the lid (2), which is made of wood, preferably being of natural beech, although it can also be of synthetic material. The wood or synthetic material are elements that do not enable the fragrance to be released to the outside in a natural way, therefore and for said purpose, a through bore or hole (20) is made, preferably circular in order to facilitate the production thereof, which is filled with an absorbent filter (3) made up of synthetic and ecological material. In the inner contour of the lid, there is a threading area (5) to be defined below.

Furthermore, as can be seen in Figure 4, the filter (3) must fit in the gap of the bore, therefore due to this bore (30) being preferably circular, the filter (3) generally has a cylindrical shape.

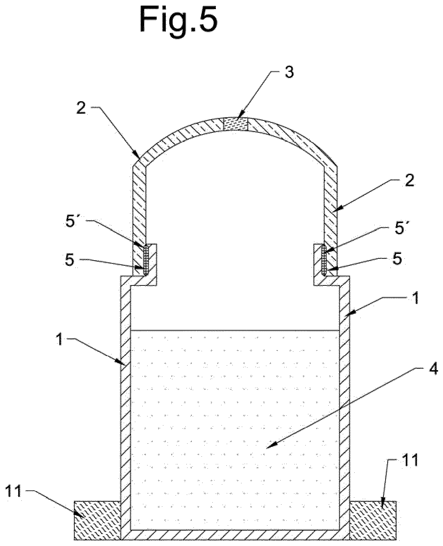

Lastly, as can be seen in the cross section of Figure 5, the assembly of the air freshener device is made up of the bottle (1) closed by threading with a lid (2) with a central through hole (20) filled with an absorbent filter (3). A detailed view of the filter (3) shows that it is not in direct contact with the liquid or fragrance (4). In turn it can be seen that the closure between the lid and the bottle is by threading, and therefore, at the upper perimeter of the bottle and the inner face of the lid, there is an inner and outer thread (5, 5') that enables the advance, coupling and closure between both elements. The thickness of the lid, and the diameter of the central hole, may be variable. This cross section shows the disk support (11) made of wood, which is an accessory, located in the lower area of the assembly. Although it is not shown, the device can be hung with the insertion in the lid of a hooking means to a rope, string or similar, although the nature of the present invention, as can be seen in these drawings, is to be placed on a surface.

Having sufficiently described the nature of the invention above, taking into account that the terminology that has been used in this specification should be taken in a broad and non-limiting sense, as well as the description of the best mode of carrying it out in practice, it is requested that the patent be registered, as it has been proven that it constitutes a positive technical development, the essence of said invention being what is specified below in the following claims.

## Claims

1. An air freshener device for interiors made up of a bottle (1) closed by a lid (2), wherein the liquid or fragrance (4) is inside the bottle (1), wherein the liquid can be refilled, in order to be placed on a piece of furniture or hung, and which is **characterised in that** the bottle (1) is made of transparent blown glass and the lid (2) has a central and circular through bore or hole (20) that is filled with an absorbent filter (3) made of synthetic and ecological material that is soaked by a tilting action of the device, wherein at the upper perimeter of the bottle (1) and the inner face of the lid (2) there is an inner and outer thread (5, 5') for a secure closure of the assembly by threading.

2. The air freshener device for interiors according to the characteristics of claim 1, wherein the lid (2) is **characterised in that** it is made of natural beech wood.

3. The air freshener device for interiors according to the characteristics of claim 1, wherein the lid (2) is **characterised in that** it is made of synthetic material.

4. The air freshener device for interiors according to the characteristics of claim 1, wherein the filter (3) is **characterised in that** it has a cylindrical shape.

5. The air freshener device for interiors according to the characteristics of claim 1, wherein the lid (2) is **characterised in that** it has an outer perimeter groove or slot (21).

6. The air freshener device for interiors according to the characteristics of claim 1, wherein the bottle (1) is **characterised in that** it has a disk support (11) made of wood in the outer lower portion thereof.
